Europäisches Patentamt

European Patent Office

Office européen des brevets

Publication number: **0 290 062**

**A2**

# EUROPEAN PATENT APPLICATION

(21) Application number: 88200589.5

(22) Date of filing: 29.03.88

(51) Int. Cl.4: **A61M 16/00**

---

The title of the invention has been amended (Guidelines for Examination in the EPO, A-III, 7.3).

(30) Priority: 03.04.87 NL 8700790

(43) Date of publication of application:
09.11.88 Bulletin 88/45

(84) Designated Contracting States:
AT BE CH DE ES FR GB GR IT LI LU NL SE

(71) Applicant: **STICHTING PEDIATRIC ARTIFICIAL RESPIRATION (S.P.A.R.)**
**Keizersgracht 207**
**NL-1016 DS Amsterdam(NL)**

(72) Inventor: **Boelens, Martinus**
**Frans Halsstraat 14**
**NL-1506 LG Zaandam(NL)**
Inventor: **Deen, Louis**
**Keizersgracht 207**
**NL-1016 DS Amsterdam(NL)**

(74) Representative: **Flamman, Han**
**LIOC Patents and Licensing P.O. Box 54**
**NL-3970 AB Driebergen(NL)**

---

(54) **High-frequency jet ventilation apparatus.**

(57) Apparatus for easing, supporting or completely taking over a patient's respiration. An air and gas mixture is fed intermittently at a frequency of approximately 100 to 800 pulses per minute, through a tubular inlet into a chamber, from where it is conducted also intermittently, into the respiratory system of the patient via at least one tubular outlet which is connected to the said respiratory system. To that end, the mouth of the inlet is situated close to the outlet. The chamber further has a second outlet, from where during the phase of expiration of the respiratory cycle may escape air/oxygen, nearly without experiencing any resistance. By means of specific adaptations, the apparatus may be used in combination with 'continuous positive airway pressure'-apparature or with ventilation apparature.

FIG.2

## Apparatus for easing, supporting or completely taking over a patient's respiration with the aid of high-frequency ventilation.

The invention relates to apparatus for easing, supporting or completely taking over a patient's respiration with the aid of an air/oxygen mixture fed intermittantly at a certain frequency of approximately 100 to 800 pulses per minute to the patient's respiratory system and which comprises a tubular portion with an internal diameter of approximately 1.5 to 3.0 mm. and through which the intermittant gas stream is fed.

Various methods and the associated apparatus are known for easing, supporting or completely taking over a patient's respiration. As an example the so-called CPAP (Continuous positive airway passage) is known. With this system, an oxygen/air mixture is conducted by a small diameter tube into air passage of a spontaneously breathing patient via the nostrils.
The gas flow is continuous and through which the respiration is eased and deepened and the greater than usually normal pressure-difference arising herefrom in the lungs leads to an essential supporting of the patient's respiration. The apparatus needed for the practice of this method is essentially simple and comprises an air/oxygen mixer, a gasflow meter, a pressure safeguarding device, a humidifier and finally a chamber from which the mixture is conducted to the patient's nose via a small diameter tube.

When the quoted CPAP method does not produce the desired result, artificial respiration is often applied. The principle of operation of this system is that a gas/air mixture is intermittantly fed to the patient at a frequency of 5 to 100 pulses per minute. To this end, the gas-stream is conducted to the patient's lungs via the intubation into the patient's trachea of a supple endotrachial tube with an internal diameter of approximately 2.5 mm. and a length of 120 mm. Thereafter, a certain pressure is built up in the lungs . It is then ensured that when a certain predetermined pressure is reached, the pressure caused by the gas-stream falls or nearly falls away. This marks the termination of the inhalation phase and after which the inspiration phase begins and through which the patient spontaneously expirates. In certain cases however, this artificial ventilation gives problems which can traced back principally to the relatively high-pressure arising during the inspiration phase.

In 1967 when the HFJV (high-frequency jet ventilation) system was developed, the patients breathing system was conneted to a high-frequency (approximately 100 to 800 pulses per minute) flow of an air/oxygen mixture. This was achieved by feeding a narrow tube with a diameter of approximately 1.5 to 2 mm through an endotracial tube with an internal diameter of approximately 11 mm. and thereafter feeding the stream of gas therethrough at a high rate.

Due to the great difference between the the two tubes, the patient can expirate through the wider tube with little resistance. This method can also be applied in combination with normal respiration.

HFJV appears particularly suitable for application with newly born babies, and particularly in the case of the prematurely born. If the baby itself can breath, its breathing will be more favourably supported by HFJV than by conventional respiration; on one hand because the inspiration flow of HFJV which closely approaches that of the infant and on the other hand because the child has no need to "fight" against the ventilator as indeed occurs with conventional respiration.

Application of HFJV to such infants does however lead to problems. This has its origin in the fact that the internal diameter of the endotrachial tube can only be 2.5 mm diameter if it is to be inserted into the trachea of a newly born baby. The insertion of a HFJV supply tube which must have a diameter at the minumum of 1.5 mm. into the endotrachial tube leads to it being necessary to overcome too high an air resistance on expirating. A solution to this problem in the direction of utilising a venturi system or using a double tube does not give the correct result.

The object of the invention is provide an apparatus which enables HFJV to be applied to new born babies whereby free expiration is possible.

According to the invention, the apparatus exhibits the characteristic that the end of the tubular portion emerges into a chamber which incorporates at least one substantially tubular outlet by which means it can be connected to a patient's respiratory system and which has at least one second outlet, in which the emerging end of the tubular portion lies close to the first outlet/ outlets of the chamber running at least mainly coaxial therewith at that position.

The tube through which the high-frequency pulsed mixture is fed now no longer needs to be inserted into the endotrachial tube but is conducted to a chamber which is indeed connected to the patient's bronchial passages via a first outlet and which is further provided with an orifice to the exterior of the chamber. This orifice is preferably located perpendicular to the JVJF jet-stream and has a relatively larger diameter of 15 mm. for example. Due to the fact that the end of the supply tube for the stream of HFJV emerges close to the first outlet and lies at that position at least co-axial therewith. the gas pulses will propagate in a fairly unchanged manner via the first outlet to the patient's lungs.

When such apparatus is used for the supply of a HF stream via a patient's nose for example it will be of advantage sometimes to provide the chamber with two first outlets each of which is connected to the patient's nose by a tube.

In order to guarantee free expiration by the patient, care must be taken to ensure that the cross-sectional area of the gas passage at the point where the first outlet or outlets respectively are positioned are sufficiently large: that is to say nowhere therealong less than approximately 8 mm$^2$.

An exemplary embodiment of the apparatus according to the invention which can be used in combinationwith CPAP installation is characterised in that it has a cylindrical form in which the wall of the chamber is provided on one end with two first outlets in the form of two outwardly extendin tubes, in which the other end of the chamber forms a second outlet and in which the side wall converges conically whilst the tubular portion connects to the CPAP installation via the side wall.

When such apparatus is combined with a CPAP installation, the patient can breathe freely in and out only when CPAP is used, or can obtain an HF gas stream alone or can be supplied from a combination of both.

Another embodiment of the apparatus according to the invention exhibits the characteristic that the chamber is approximately cylindrical in form, in which the side wall converges conically to form the first outlet which is suitable for intubation into the patient's endrotrachial tube, in which the second outlet is located in the non-conical portion of thechamber's side wall and in which the other end of the tubular portion extends into the chamber at least so far therein that the emerging end thereof lies past the second outlet and preferably in the conicallyformed portion.

This embodiment is particularly suitable for intubation into the trachea. Since the supply tube for the HF stream runs into the conical portion, there will be extra promotion of gas-pulse propagation to the trachea due to the conical form of the side wall. In any case this requires the supply tube to extend into the chamber past the second outlet in order to prevent leakage and turbulation.

The second outlet can be provided with a connection for a ventilating installation in combination with which the apparatus according to the invention can be used or can be used either for administering a HF gas stream only or for administering a HF gas stream in combination with conventional breathing or for conventional breathing only.

The invention comprises both an appliance in which the apparatus according to the invention is combined with a CPAP installation or an appliance in which it is combined with a conventional ventilator installation or an appliance in which both appliances are incorporated.

The invention is now to be described further with reference to the accompanying drawings in which:

Figure 1 shows a schematical cross-section of an apparatus according to the invention which is suitable for use in combination with CPAP.

Figure 2 shows a schematical cross-section of apparatus according to the invention which is suitable for use in combination with a conventional ventilator and,

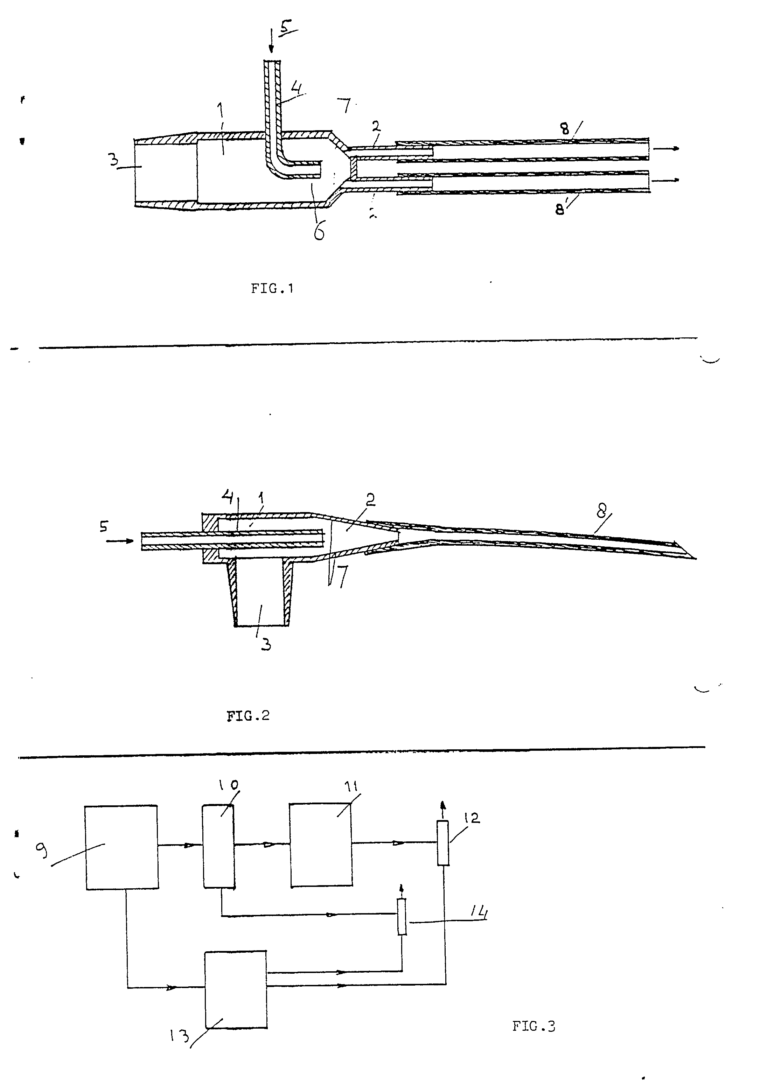

Figure 3 shows schematically an appliance which can be used with CPAP, a conventional ventilator, with HFJV or a combination of HFJV with one of the first two quoted appliances. Fig. 1 shows a chamber provided with two first outlets 2 and 2' and second outlet 3. An end of the tubular portion 4 emerges into the chamber and through which the high-frequency gas stream 5 is led into the chamber. Tubes 8 and 8' are affixed to outlets 2 and 2' respectively and are guided to the patient's bronchial passages via the nose. The end 6 of the tube 4 lies near to the outlets 2 and 2' and in which position the tube 4 and the outlets 2 and 2' lie coaxial to one another so that gas pulses led into the chamber via the tube 4 can be propagated via the outlets 2 and 2' and the tubes 8 and 8' to the patient's bronchial passages.

The patient can expirate freely along the tubes 8 and 8' via the relatively large outlet 3. To this end care must be taken that there is no hindering restriction along the passage to the outlet 3. The cross-section of the passage of expirated gas must be at least and approximately 8 mm$^2$ therealong.

Such apparatus may be used in combination with CPAP, and to this end the second outlet 3 is connected to the CPAP installation. Thus in the interests hereof, and as shown in the drawing, the wall portions 7 of the chamber 1 converges conically toward the outlet 3.

Figure 2 shows an apparatus according to the invention used for intubation into the trachea. The high-frequency gas stream 5 is led into the chamber 1 via the tubular portion 4. The first outlet 2 on the other end of the chamber 1 is formed by the conically converging wall portion 7 thereof whilst the outlet 3 is located in the side wall of the chamber 1. In order to ensure that the gas pulses are effectively fed from the tube 4 via the outlet 2, the tube 4 runs up the the conically converging portion 7 of the chamber 1 past the outlet 3 in order to eliminate gas leakage and turbulation on the way. The aperture between the tubular surface of the tube 4 and the wall of the chamber 1 always needs to be such that no resistance to expiration is encountered.

With the aid of an endotrachial tube 8, the outlet 2 of such an apparatus is intubated into a patient's bronchial passage. Free expiration is then always possible via the outlet 3. The apparatus according to figure 2 can be used in combination with a ventilating installation which is connected to the opening 3 and to this end the opening 3 is of conical form as shown in the drawing.

Figure 3 shows schematically an appliance with which the use of HFJV is possible in combination with CPAP or conventional ventilation or for use with each apart.

The air and oxygen is mixed in the blender 9 in the desired ratio. The mixture is fed via a a gas-flow meter 10 to a conventional ventilator 11 which is in turn connected to the second outlet of the apparatus 12 as shown in figure 2 and which is also connected to a HFJV unit by its tubular portion 4, see figure 2. Thus via the first outlet of the apparatus 12, conventional ventilation, HF ventilation or a combination of both can be resorted to.

All units working with the appliance can be changed over from one method of operation to another or arranged to work with a certain combination thereof.

The appliance according to figure 3 also incorporates a CPAP installation. The gas mixture is fed via the flowmeter 10 to the second outlet 3 from an apparatus 14 as drawn in figure 1. This apparatus 14 is also connected to the HFJV unit 13 via its tubular portion 4 of figure 1.

## Claims

1. Apparatus for easing, supporting or completely taking over a patient's respiration with the aid of an air and gas mixture fed intermittantly at a frequency of approximately 100 to 800 pulses per minute to the patient's respiratory sustem and which comprises a tubular portion with an internal diameter of approximately 1.5 to 1.2 mm. and through which the intermittant gas stream is fed and characterised in that the end of the tubular portion emerges into a chamber which has at least one outlet of approximately tubular form and by which means it can be connected to the patient's respiratory system and which has at least a second outlet and in which the mouth of the tubular portion lies near to the first outlet or outlets from the chamber and which runs substantially co-axial therewith at this position.

2. Apparatus as claimed in claim 1 characterised in that the cross-sectional area of the gas passage between the first outlet or outlets and the second outlet must not be less than 8 mm$^2$ anywhere.

3. Apparatus as claimed in claim 1 or claim 2 used in combination with a CPAP (continuous positive airway pressure) installation and characterised in that the chamber is approximately cylindrical in form, in which the wall is provided at one end with two first outlets in the form of outwardly extending tubes, in which the other end forms the second outlet and in which the side wall converges conically whilst the tubular portion is led thereinto for connection to the CPAP installation via the side wall.

4. Apparatus as claimed in claim 1 or claim 2 characterised in that the chamber is approximately cylindrical in form, in which the side wall thereof converges conically to form a first outlet which is suitable for intubation into a patient's bronchial passages with the aid of an endotracheal tube coupled to the first outlet, in which the second outlet is located on the non-conical part of the side wall and in which the tubular portion extends into the non-conical side wall of the chamber at least so far that the emerging end thereof lies past the second outlet and preferably in the conical portion.

5. Apparatus as claimed in claim 4 for use in combination with a ventilating installation and characterised in that the second outlet forms a connection for a ventilating installation.

6. Appliance for easing, supporting or completely taking over a patient's respiration comprising a ventilating installation combined with an apparatus as claimed in claim 5.

7. Appliance for easing, supporting or completely taking over a patient's respiration comprising a CPAP installation combined with an apparatus as claimed in claim 3.

8. Appliance for easing, supporting or completely taking over a patient's respiration in which both the appliances claimed in claim 6 and claim 7 are incorporated.

0 290 062

FIG.1

FIG.2

FIG.3